# EUROPEAN PATENT APPLICATION

(11) **EP 3 461 505 A1**
(43) Date of publication of application: **03.04.2019**
(21) Application number: 18194943.9
(22) Date of filing: 17.09.2018
(51) Int. Cl.: A61L 27/10, A61L 27/24, A61L 27/36, A61L 27/54, A61L 27/12, A61L 27/46, A61L 24/00, A61L 24/10

(54) **COAGULUM-BASED BIOMATERIAL COMPOSITIONS AND METHODS THEREOF**

(30) Priority: 27.09.2017 US 201762563685 P; 07.09.2018 US 201816124315
(71) Applicant: Globus Medical, Inc., Audubon, PA 19403 (US)
(72) Inventor: BHAT, Archana, PHOENIXVILLE, PA Pennsylvania 19460 (US); DAVIS, Madeline, MALVERN, PA Pennsylvania 19355 (US)
(74) Representative: Morabito, Sara

(57) **Abstract**

Biomaterials, implants made therefrom, methods of making the biomaterial and implants, methods of promoting bone or wound healing in a mammal by administering the biomaterial or implant to the mammal, and kits that include such biomaterials, implants, or components thereof. The biomaterials may be designed to exhibit osteogenic, osteoinductive, osteoconductive, and/or osteostimulative properties.

## Description

### TECHNICAL FIELD

The present invention relates generally to bone and tissue healing biomaterials, and in particular, coagulum-based compositions for regeneration of bone or cartilage. The invention also relates to methods of making the materials and implants, and methods of promoting bone or wound healing in a mammal by administering the biomaterial or implant to the mammal. The invention further relates to kits that include one or more of the biomaterials, implants, or components thereof.

### BACKGROUND

Bone grafting is a surgical procedure that replaces missing bone and/or repairs bone fractures. Bone generally has the ability to regenerate well but may require a scaffold to do so. Grafts may be allograft (e.g., cadaveric bone from a bone bank), autologous (e.g., bone or tissue harvested from the patient's own body), or synthetic. Most bone grafts are expected to be resorbed and replaced as the natural bone heals over time.

Successful biomaterials may include osteoconduction (guiding the reparative growth of the natural bone), osteoinduction (encouraging undifferentiated cells to become active osteoblasts), and/or osteogenesis (living bone cells in the graft material contributing to bone remodeling). Although traditional grafts may exhibit certain advantages, traditional allograft may not exhibit the properties desired, may be difficult to obtain, or may not be in a shape or form suitable for implantation.

### SUMMARY

To meet this and other needs, coagulum-based compositions and biomaterials described herein may be osteogenic, osteoinductive, osteoconductive, and/or osteostimulative, which may be advantageous for tissue, bone, or cartilage healing and repair and without the drawbacks of present allograft or autograft products. The coagulum-based compositions or implants prepared therefrom can include various combinations of coagulum (e.g., derived from bone marrow aspirate and/or blood), an exogenous recombinant protein such as BMP-2 or BMP-6, ceramics such as tricalcium phosphate, bioactive glass, and combinations thereof, collagen fibers, and/or one or more additional components each of which is described in more detail herein.

According to one embodiment, a composition for aiding tissue regeneration includes a coagulum comprising a hematic fluid; an exogenous recombinant protein; and ceramic particles, collagen fibers, or both. According to another embodiment, a composition for aiding tissue regeneration includes a scaffold comprising one or more of ceramic particles, collagen fibers, and mixtures thereof; a coagulum comprising a hematic fluid dispersed through the scaffold; and an exogenous recombinant protein.

The coagulum-containing compositions may include one or more of the following features. The coagulum may include a bodily fluid or tissue derived from a patient, which comprises one or more of platelets, red blood cells, white blood cells, plasma, bone marrow stroma, mesenchymal stem cells, osteoprogenitor cells, myelopoietic cells, erthyropoietic cells, and combination thereof. The coagulum may include viable cells, for example, obtained from the patient. The coagulum may be coagulated bone marrow aspirate, coagulated blood, or a mixture thereof. The recombinant protein may be a bone morphogenetic protein, such as BMP-2, BMP-4, BMP-6, BMP-7, heterodimers thereof, and combinations thereof. The ceramic particles may include bioactive glass, tri-calcium phosphate, and mixtures thereof. The ceramic particles may range in size from about 1 to 1000 µm and/or the ceramic particles may have a pore size of about 100 to 500 µm with an overall porosity of about 60-80%.

According to yet another embodiment, a method of preparing an implantable composition for aiding tissue regeneration may include obtaining a sample of hematic fluid from a patient without using an anti-coagulant to withdraw the sample; mixing the sample with an exogenous recombinant protein and ceramic particles, collagen fibers, or both to form a homogenous mixture; and allowing the homogenous mixture to naturally coagulate without adding a coagulation agent to form the implantable composition. The recombinant protein and ceramic particles, collagen fibers, or both may be mixed with the hematic sample before the sample coagulates. The implantable composition may be administered to the same patient from which the sample was obtained.

According to another embodiment, a method of promoting bone or wound healing in a mammal may include providing a composition comprising a mixture of coagulum comprising a hematic fluid, an exogenous recombinant protein, and ceramic particles, collagen fibers, or both; and administering the composition into a target repair site to facilitate repair or regeneration of tissue at the target repair site. The target repair site may be an orthopedic injury or defect, for example, in the spine. The tissue being regenerated may be bone, cartilage, or other tissue.

According to yet another embodiment, a kit includes one or more of the components, compositions, or implants described herein, blood or bone marrow aspirate retrieval kits, and trays, syringes, or other components for combining and administering the blood or bone marrow aspirate with the other biomaterial components. For example, the kit may contain powder, putty, gel, strip, and/or extrudable versions of the compositions. The kit may contain compositions of the same or different types. In addition, the kit may include other components known in the art, including, but not limited to, carriers or scaffolds, cages (e.g., titanium and/or polyether ether ketone (PEEK) spacers), allograft spacers, cell culture media, phosphate buffered saline (PBS), a tissue culture substrate, retrieval tools, harvesting tools, implantation tools, or the like.

According to a further embodiment it is provided a composition for aiding tissue regeneration, the composition comprising: a scaffold comprising one or more of ceramic particles, collagen fibers, and mixtures thereof; a coagulum comprising a hematic fluid dispersed through the scaffold; and an exogenous recombinant protein.

### DETAILED DESCRIPTION

The present invention relates generally to coagulum-based biomaterials and implants made therefrom that may exhibit osteogenic, osteoinductive, osteoconductive, and/or osteostimulative properties. The invention also relates to methods of making the compositions and implants, and methods of promoting bone or wound healing in a mammal by administering the biomaterial or implant to the mammal. The invention further relates to kits that include one or more of the biomaterials, implants, retrieval kits for obtaining bone marrow aspirate and/or blood, tools and trays for mixing and combining ingredients, and other components thereof.

Additional aspects, advantages and/or other features of example embodiments of the invention will become apparent in view of the following detailed description. It should be apparent to those skilled in the art that the described embodiments provided herein are merely exemplary and illustrative and not limiting. Numerous embodiments of modifications thereof are contemplated as falling within the scope of this disclosure and equivalents thereto.

In describing example embodiments, specific terminology is employed for the sake of clarity. However, the embodiments are not intended to be limited to this specific terminology. Unless otherwise noted, technical terms are used according to conventional usage.

As used herein, "a" or "an" may mean one or more. As used herein "another" may mean at least a second or more. As used herein, unless otherwise required by context, singular terms include pluralities and plural terms include the singular.

As used herein and in the claims, the terms "comprising" and "including" are inclusive or open-ended and do not exclude additional unrecited elements, compositional components, or method steps. Accordingly, the terms "comprising" and "including" encompass the more restrictive terms "consisting essentially of" and "consisting of."

Unless specified otherwise, all values provided herein include up to and including the endpoints given, and the values of the constituents or components of the compositions are expressed in volume percent or % by volume of each ingredient in the composition.

Each compound used herein may be discussed interchangeably with respect to its chemical formula, chemical name, abbreviation, etc. For example, BMP may be used interchangeably with bone morphogenic protein.

Embodiments described herein may be generally directed to coagulum-based compositions, implants made therefrom, methods of making the same, and methods of using the same to promote healing of tissue and/or fusion of bone. Although biomaterials or implants may be discussed separately, it will be appreciated by one of ordinary skill in the art that the biomaterials described may be used in and of itself or may be used to create implants of different shapes, sizes, and orientations for a number of different clinical outcomes. Thus, the discussion of biomaterials may apply equally to the discussion on implants and vice versa.

The coagulum-based compositions may be osteogenic, osteoinductive, osteoconductive, and/or osteostimulative, which may be advantageous for tissue healing and repair. The biomaterials may be osteoconductive when the material serves as a scaffold that provides surface area for new bone growth. The biomaterials may be osteoinductive if they stimulate osteoprogenitor cells or induce mesenchymal stem cells to differentiate into osteoblasts that then begin new bone formation. Biomaterials may be osteogenic if they contain cells (e.g., viable cells) that are capable of bone regeneration. The biomaterial may be osteostimulative if the material accelerates the bone formation process. The composition may also be "biocompatible" as that term refers to the ability (e.g., of a composition or material) to perform with an appropriate host response in a specific application, or at least to perform without having a toxic or otherwise deleterious effect on a biological system of the host, locally or systemically. The biomaterial and/or implant or a portion thereof may be "biologically degradable" in that the material may be degraded by cellular absorption and/or hydrolytic degradation in a patient's body.

The coagulum-based compositions may contain an exogenous recombinant protein, namely, recombinant proteins not already present in the coagulum. Delivery of recombinant growth factors is a challenge due to lack of appropriate binding sites in the scaffold. This lack of appropriate binding sites may cause a rapid release of the growth factors at the time of implantation and may lead to excessive bone formation, both at the implantation site and in surrounding tissues. The use of a blood or bone marrow aspirate (BMA) derived coagulum provides necessary binding sites for the recombinant protein (e.g., bone morphogenic proteins (BMPs)), and additionally provides osteoprogenitor cells, mesenchymal stem cells, and/or hematopoetic cells that aid in bone and tissue regeneration.

The coagulum-based compositions provide an autologous or allogenic carrier to deliver recombinant proteins for treating bone or cartilage regeneration. By adding additional osteoconductive and/or osteostimulative components to the coagulum, the regeneration process can be further mediated with mechanical support and cell attachment sites. The controlled release of the recombinant protein will help to reduce the side-effects previously seen with this type of technology.

According to one embodiment, the coagulum-based compositions may be configured to facilitate repair or regeneration of tissue, for example, bone, cartilage, or other tissue. In particular, the coagulum-based compositions may facilitate repair or regeneration of tissue at a target repair site. The target repair site can be, for example, a void, gap, or other defect, or a surgeon created opening in bone, cartilage, between bones, or other structure or tissue location in a body of a patient. For example, the biomaterial composition can be configured to facilitate bone growth at a target repair site in the spine, pelvis, an extremity, the cranium, or another bone, between bones, or bony structure in the patient's body. The biomaterial composition may also be configured to facilitate cartilage growth at a target repair site. The biomaterial composition may be configured to be directly implanted or otherwise disposed at and in contact with the target repair site. The patient and target repair site may be in a human, mammal, or other organism.

The biomaterial compositions can include various combinations of coagulum including coagulum derived from blood and/or bone marrow aspirate; exogenous recombinant protein including bone morphogenic proteins; ceramic such as calcium phosphate or bioactive glass; collagen fibers; and/or one or more additional components each of which is described in more detail herein.

The biomaterial composition may include a coagulum component. The coagulum is a coagulated hematic fluid. The hematic fluid may include blood-containing or blood-derived fluids, for example, withdrawn or obtained from a patient. In an exemplary embodiment, the coagulum may be a coagulated bone marrow aspirate, a coagulated blood, or a coagulated blood-based component (e.g., coagulated plasma). The bone marrow aspirate may include bone marrow tissue obtained from the interior of bones or a component thereof. The blood may include whole blood, peripheral blood, or a component thereof. The blood may be obtained, for example, from the veins of a patient. The hematic fluid is preferably withdrawn from the patient without an anticoagulant, for example, without an intravenous anticoagulant. Before or after being mixed with a scaffold component (e.g., the ceramic and/or collagen fibers), the hematic fluid is permitted to coagulate by the natural process of coagulation. In other words, no additional coagulating factors or agents are applied to the hematic fluid to induce or promote coagulation.

Coagulation may also be referred to as clotting and is the process by which the hematic fluid changes from a liquid to a gel, thereby forming a coagulant. Coagulation involves both a cellular component (e.g., platelet) and a protein component (e.g., coagulation factors or clotting factors), which are naturally present in the hematic fluid. Coagulation may begin almost instantly after the blood or bone marrow aspirate is retrieved from the patient. For example, autologous or allogenic whole blood or BMA collected without the anticoagulant has a clotting time of approximately 15s to 10min. The scaffold component (e.g., the ceramic and/or collagen fibers) may be mixed with the hematic fluid before or during this time to produce the coagulum. The coagulum mixture may be used immediately in the patient and applied at the surgical site, for example, for tissue healing. Alternatively, the coagulum mixture may be packaged and stored for later use.

The coagulum is a coagulated hematic fluid. Preferably, the hematic fluid and resulting coagulum is minimally manipulated such that the native cells and components of the hematic fluid remain in the resultant coagulum. The hematic fluid and/or the resulting coagulum may include one or more of platelets, red blood cells, white blood cells, plasma, bone marrow stroma, mesenchymal stem cells, osteoprogenitor cells, myelopoietic cells, erthyropoietic cells, and combination thereof. The coagulum may include viable cells, and preferably includes native viable cells from the patient. In other words, the coagulum is preferably minimally manipulated such that native viable cells are not damaged or harmed during processing and the native cells remain viable when implanted into the patient.

When bone marrow aspirate is the hematic fluid, the hematic fluid may include one or more of red blood cells, platelets, white blood cells, bone marrow stem cells, marrow adipose tissue, stroma, trabecular bone, blood-clotting factors, fibroblasts, macrophasges, adipocytes, osteoblasts, osteoclasts, endothelial cells, mesenchymal stem cells, and other cells and tissues of bone marrow. The bone marrow may include red marrow made of primarily hematopoietic tissue and/or yellow marrow made of primarily fat cells. The marrow may be harvested from flat bones, such as the pelvis, sternum, cranium, ribs, vertebrae and scapulae, in the cancellous bone at the epiphyseal ends of long bones, such as the femur and humerus, and in the intramedullary cavity of long and short bones.

When blood is the hematic fluid, the hematic fluid may include one or more of thrombocytes (plasma), erythrocytes (red blood cells), leukocytes (white blood cells), platelets, serum albumin, blood-clotting factors, immunoglobulins, lipoprotein, electrolytes, and other cells and tissues of blood. The blood may include peripheral blood, which is found within the circulating pool of blood and not sequestered within the lymphatic system, spleen, liver, or bone marrow. The blood may be withdrawn from the vein of a patient. In the event that an allogenic source is used, the blood type of the hematic fluid will be determined and the hematic fluid will be crossmatched according to conventional techniques to ensure that a compatible hematic product is used in the patient.

The hematic fluid may coagulate to form the coagulum according to natural pathways. The hematic fluid naturally contains the clotting factors necessary for the hematic fluid to coagulate without the addition of an exogenous clotting factor. The coagulation factors may include one or more of the following factors, but are not limited to, Factors I (fibrinogen), II (prothorombin), III (tissue factor), IV (calcium), V (proaccelerin), VII (stable factor), VIII (antihemophilic factor A), IX (antihemophilic factor B), X (Stuart-Prower factor), XI (plasma thromboplastic antecedent), XII (Hageman factor), XII (fibrin-stabilizing factor), von Willebrand factor, prekallikrein, high molecular weigth kinninogen (HMWK), fibronectin, antithrombin III, heparin cofactor II, protein C, protein S, protein Z, protein Z-related protease inhibitor (ZPI), plasminogen, alpha 2-antiplasmin, tissue plasminogen activator (tPA), urokinase, plasminogen activator inhibitor-1 (PAI1), plasminogen activator inhibitor-2 (PAI2), and cancer procoagulant. Although the clotting factors may be naturally present in the hematic sample, it is envisioned that an exogenous clotting factor may be used if desired to enhance or expedite coagulation.

The hematic fluid (before coagulation) and/or coagulum (after coagulation) may be present in the final composition as a primary component. In other words, the hematic fluid and/or coagulum may be present in an amount greater than 50% v/v. More particularly, the hematic fluid and/or coagulum may be present in a range of about 50-90% v/v, about 50-80% v/v, about 60-80% v/v, about 60-70% v/v, or about 62-68% v/v. By way of example, 5cc of hematic fluid or coagulum may be mixed with 2-3cc of the scaffold component(s).

The compositions may include an exogenous recombinant protein component. The hematic fluid may contain one or more naturally occurring recombinant proteins. The composition may include an exogenous recombinant protein added to the composition. The recombinant protein component may comprise a bone morphogenetic protein. The bone morphogenetic protein may include BMP-2, BMP-4, BMP-6, BMP-7, heterodimers thereof, and combinations thereof. The blood-based proteins of the coagulum provide several receptors that bind to recombinant proteins, such as BMP-2, BMP-4, BMP-6, and BMP-7. This may cause the recombinant protein to be released in a controlled-fashion once implanted. The recombinant protein may be mixed with the blood or BMA prior to coagulation to ensure homogenized distribution throughout the coagulum. The dosage rate of recombinant protein may be, for example, on the order of about 25 µg/cc - 1.5 mg/cc of coagulum.

According to certain embodiments, the compositions may include a ceramic component, for example, as a scaffold component. For example, the ceramic may include ceramic mineral or inorganic filler useful for promoting bone formation. The ceramic component may include, but is not limited to, synthetic and naturally occurring inorganic fillers such as alpha-tricalcium phosphate, beta-tricalcium phosphate, tetra-tricalcium phosphate, dicalcium phosphate, calcium carbonate, barium carbonate, calcium sulfate, barium sulfate, hydroxyapatite (HA), biphasic calcium phosphate (e.g., composite between HA and β-TCP), bioactive glass, and combinations and mixtures thereof. Tricalcium phosphate and bioactive glass share similar surface properties and show enhanced osteoconductivity in *in vivo* settings. Tricalcium phosphate has a similar composition to hydroxyapatite, but resorbs faster due to a lower calcium to phosphate (Ca/P) ratio. For example, hydroxyapatite has a Ca/P ratio of about 1.67 whereas tricalcium phosphate has a Ca/P ratio of about 1.5.

The ceramic may be added to the mixture prior to coagulation. The ceramic component may provide osteoconductive and osteostimulative components to the construct by providing cell attachment sites and stimulating osteoblast proliferation and differentiation. The porosity, pore size, and pore geometry of the ceramic component can be tailored for the specific tissue regeneration application. In the case of bone tissue regeneration, a pore size of 100-500µm may be targeted with an overall porosity of 60-80%. The ceramic may also exhibit a longer resorption time *in vivo* than the coagulum, therefore providing mechanical support during the later stages of bone regeneration.

If present, one or more ceramics may be included in the composition depending on the type or types of ceramic present, for example, in amounts ranging from about 5-50% v/v, about 10-50% v/v, about 20-50% v/v, about 10-40% v/v, about 20-40% v/v, about 30-40% v/v, about 20-30% v/v, about 25-35% v/v, or about 30-35% v/v. By way of example, 5cc of hematic fluid or coagulum may be mixed with about 2.5-3cc of ceramic.

In certain embodiments, the ceramic comprises beta-tricalcium phosphate (TCP). The calcium phosphate may be configured to facilitate regrowth of bone at the target repair site. In some embodiments, the calcium phosphate of the bone graft composition is an osteoinductive agent. The calcium phosphate is configured to be mixed within, disposed on, embedded in, or otherwise combined with the biomaterial composition. The calcium phosphate can be in any suitable form. For example, the calcium phosphate can be in particulate or granular form. The calcium phosphate may have a particle size ranging from about 1 to 500 µm, about 25 to about 450 µm, about 50 to about 400 µm, about 75 to about 300 µm, or about 100 to about 250 µm. The calcium phosphate may be porous or non-porous.

The ceramic may also comprise a bioactive glass. The bioactive glass may also be configured to facilitate the regrowth of bone at the target repair site. In some embodiments, the bioactive glass can be an osteoconductive agent. Bioactive glass possesses osteostimulative properties, which may be useful in the regeneration of hard tissues. The bioactive glass can be disposed on, embedded within, and or mixed within the biomaterial composition. The bioactive glass can be any alkali-containing ceramic, glass, glass-ceramic, or crystalline material that facilitates bone formation after contact with a biological environment. Suitable bioactive glasses include sol gel derived bioactive glass, melt derived bioactive glass, silica based bioactive glass, silica free bioactive glass such as borate based bioactive glass and phosphate based bioactive glass, crystallized bioactive glass (either partially or wholly), and bioactive glass containing trace elements or metals such as copper, zinc, strontium, magnesium, zinc, fluoride, mineralogical calcium sources, and the like.

Exemplary bioactive glass can include bioglass 45S5 (46.1 mol% SiO₂, 26.9 mol% CaO, 24.4 mol% Na₂O and 2.5 mol% P₂O₅), 58S (60 mol% SiO₂, 36 mol% CaO and 4 mol% P₂O₅), 70S30C (70 mol% SiO₂, 30 mol% CaO), or a combination of the foregoing bioglass. The bioactive glass may take the form of fibers, granules, particles, or a combination thereof. The bioactive glass may be irregular in shape, for example. The bioactive glass may have a unimodal or bimodal particle size distribution. The bioactive glass may have a particle size, for example, ranging from about 1 to 1000 µm, about 50 to 750 µm, or about 75 to 500 µm. Particle size and distribution may be determined by routine techniques known in the art including sieve analysis or BET (Brunauer, Emmett and Teller) testing, for example. The bioactive glass particles may have a pore size of about 100 to 500 µm with an overall porosity of about 60-80%.

According to certain embodiments, the compositions may include collagen. Collagen may be added for supplementary short-term scaffolding during the coagulum formation process. The collagen may have osteoconductive properties, for example, to function as a scaffold at the target repair site. In addition to providing cell attachment sites, the collagen may improve the overall handling of the coagulum with added resilience and structure. The type of collagen added can be specific to the tissue type being regenerated. The collagen can be or include soluble collagen, insoluble collagen, or a combination thereof. The collagen can be or include type I collagen, type II collagen, type III collagen, type VII collagen, another suitable type of collagen, or a combination thereof. The collagen can be derived from human, equine, bovine, porcine, murine, synthetic, or from another suitable source. In one embodiment, the collagen is of mammalian origin, preferably human. The collagen may be in fiber, particulate, gel, or another suitable form. The collagen may be porous or non-porous. In an exemplary embodiment, the collagen is in the form of fibers.

If present, collagen may be included in the composition, for example, in amounts ranging from about 1-20% v/v, about 1-10% v/v, about 5-10% v/v, or about 7-8% v/v. By way of example, 5cc of hematic fluid or coagulum may be mixed with about 0.5-1cc of collagen fibers.

According to certain embodiments, the compositions may include a bone-based material. For example, the composition may include demineralized bone matrix. Demineralized bone matrix (also known as DBM) may provide osteoconductive, osteoinductive and/or osteogenic properties. Thus, it induces the formation of bone tissue. As used herein, the terms "demineralized bone", "demineralized bone matrix", and "DBM" may be used interchangeably. The demineralized bone, for example, in the form of fibers, chips, and/or particles, can be disposed on, embedded within, and or mixed within the biomaterial composition.

Demineralized bone matrix may be in the form of sheets, fibers, threads, strips, chips, shards, elongated particles, powder, or particulates, for example. The demineralized bone matrix may include bone pieces of all shapes, sizes, thickness, and configurations that possess regular, irregular, or random geometries. For example, fibers may have an average fiber length of about 250 µm to about 2 mm, about 250 micrometers to about 750 micrometers, about 750 micrometers to about 1.25 millimeters, or about 1.25 millimeters to about 2 millimeters. In addition, the fibers may have an aspect ratio (defined as the ratio of fiber length to diameter) of about 1:1 to about 50:1, about 10:1 to about 40:1, about 5:1 to about 10:1, or about 2:1 to about 5:1. Bone chips may have a size, for example, of about 1 mm to about 10 mm, about 1mm to about 2 mm, about 1 mm to about 4 mm, about 1 mm to about 6 mm, about 2 mm to about 4 mm, about 2 mm to about 6 mm, about 4 mm to about 6 mm, about 6 mm to about 8 mm, or about 8 mm to about 10 mm across the largest dimension. Bone particles or particulates may range in size, for example, from about 0.01 to about 2 mm, about 0.1 mm to about 1.0 mm, about 100 to about 500 microns, or about 100 to about 400 microns. It will be appreciated that some variation in dimension is possible in the production of the demineralized bone materials.

In some embodiments, the bone used to manufacture the demineralized bone matrix can be cortical, cancellous, cortico-cancellous of autogenous, allogeneic, xenogeneic or transgenic in origin. Thus, the fibers, chips, or particulates, for example, can include cortical, cancellous, or cortico-cancellous bone. Preferably, the demineralized bone is in the form of fibers derived from cortical bone, powder derived from cortical bone, and/or chips derived from cortico-cancellous bone.

To prepare bone matrix, the bone material is typically treated to clean, defat, sterilize, virally inactivate, disinfect, demineralize, dehydrate, and/or dry the bone matrix. Methods for preparing DBM are known to persons of ordinary skill in the art and include, but are not limited to, shaving bone into thin shavings or fibers, milling, grinding, or crushing bone into chips or particles, or the like. Before or after processing the bone, the bone material is subjected to demineralization so as to reduce inorganic content to low levels. For example, demineralized bone can be produced by acid extraction, thermal freezing, irradiation, or physical extraction of inorganic minerals from human or animal bone. In an acid extraction, inorganic acids such as hydrochloric acid or phosphoric acid, or organic acids such as formic acid, acetic acid, peracetic acid, citric acid, propionic acid, etc. may be used. As would be recognized by one of ordinary skill in the art, the amount and depth of demineralization into the bone surface can be controlled by adjusting the treatment time, temperature of the demineralizing solution, concentration of the demineralizing solution, agitation intensity during treatment, and the like.

The term "demineralized" refers to bone or bone material containing less than its original mineral content (e.g., calcium content) and may encompass "substantially demineralized," "partially demineralized," and "completely demineralized" bone material. For example, the demineralized bone may include less than 10%, less than 9%, less than 8%, less than 7%, less than 6%, less than 5%, less than 4%, less than 3%, less than 2%, or less than 1% of the original mineral content (e.g., calcium content) of the bone.

In addition to or in place of the other scaffold(s) described herein, one or more carrier, scaffold materials, or processing additives may be used in the biomaterial composition. The carrier may affect the overall handling of the material. Preferably, the carrier is inert or enhances osteogenic, osteoinductive, osteoconductive, and/or osteostimulative properties of the composition. Suitable carriers, scaffolds, or additives may include, but are not limited to, phospholipids, carboxylmethylcellulose (CMC), glycerin, glycerol, polyethylene glycol (PEG), hydrogels, poloxamers, polylactic acid (PLA), polylactic-co-glycolic acid (PLGA), other copolymers of the same family, and combinations thereof.

Additionally, biological agents may be added to the biomaterial or implant. These biological agents may comprise a peptide, such as peptide amphiphiles with a binding affinity for BMP described by way of example in U.S. Publication No. 2016/0159869 which is incorporated by reference herein in its entirety for all purposes, a bone growth factor such as platelet derived growth factor (PDGF), vascular endothelial growth factor (VEGF), insulin derived growth factor (IDGF), a keratinocyte derived growth factor (KDGF), or a fibroblast derived growth factor (FDGF), stem cells, and platelet rich plasma (PRP), to name a few. If desired, one or more active pharmaceutical ingredients or medicaments may be incorporated into the biomaterial or implant as well. Biological agents may be added in any suitable pharmaceutically acceptable and effective amounts known in the art.

The biomaterial composition may be obtained using any suitable procedures and techniques known in the art. For example, components of the composition described herein may be mixed together to form the resulting composition. The components may be combined, for example, at room temperature (e.g., about 20 and 26 °C). The components may be mixed together before coagulation to provide for homogenous mixing. The components may be mixed in a tray or other device and may resemble the shape of the tray. The composition may also be extrudable, in liquid form, putty form, strip form, or otherwise configured for application or implantation at the surgical site.

The biomaterial and/or implant formed therefrom is intended to be applied at a bone or cartilage repair site, e.g., one resulting from injury or defect. The implant can be utilized in a wide variety of orthopedic, periodontal, neurosurgical, oral and maxillofacial surgical procedures. In particular, the biomaterials may be suitable for repairs of the vertebral column including spinal fusion and internal fixation; tumor surgery, e.g., deficit filling; discectomy; laminectomy; scoliosis, lordosis and kyphosis treatments. Possible clinical applications may include e.g., the treatment of spinal disc degeneration or disease, traumatic, pathologic, or stress fractures, congenital defects or fractures, or operative defects in any bone or between bones of the body.

The compositions and implants may be configured for use at various target repair sites within a body of a patient to facilitate bone growth therein. In some embodiments, the composition is configured for use at a target repair site in the patient's spine. For example, the composition can facilitate growth of bone between the body of a first vertebra and the body of a second vertebra to achieve interbody fusion of the two vertebrae. In a spinal fusion procedure, the composition may be used in conjunction with one or more mechanical supports (e.g., a cage or frame, spacer, plate, a plurality of screws and/or rods, or the like). Although the spine is described, the composition can be configured to be implanted into or at a target repair site in or at a different bone or bony structure of the patient's body.

The term "treating" and the phrases "treatment of a disease" and "treatment of a condition" refer to executing a protocol that may include the use of the compositions, devices and methods herein and/or administering one or more biomaterials to a patient (human, normal or otherwise, or other mammal), in an effort to alleviate signs or symptoms of the disease or condition. Alleviation can occur prior to signs or symptoms of the disease or condition appearing, as well as after their appearance. Thus, "treating" or "treatment" includes "preventing" or "prevention" of disease or undesirable condition. In addition, "treating" or "treatment" does not require complete alleviation of signs or symptoms and does not require a cure to the ailment.

Further example embodiments are directed to kits that include components for making the present biomaterials and implants, including for example, carriers or scaffolds, cages (e.g., titanium and/or polyether ether ketone (PEEK) spacers), allograft spacers, demineralized bone materials, cell culture media, phosphate buffered saline (PBS), a tissue culture substrate such as a flask, trypsin, or mixtures, bone graft harvesting tools, bone marrow aspirate retrieval tools, or the like. Additional components, instructions and/or apparatus' may also be included.

The following examples are provided to further illustrate various non-limiting embodiments and techniques. It should be understood, however, that these examples are meant to be illustrative and do not limit the scope of the claims. As would be apparent to skilled artisans, many variations and modifications are intended to be encompassed within the spirit and scope of the invention.

### EXPERIMENTAL EXAMPLES

In each of the examples described below a hematic fluid is mixed with a scaffold material. The hematic fluid is permitted to naturally coagulate to form the coagulum. Bone marrow aspirate and/or peripheral blood may be obtained from the patient by conventional means. The bone marrow aspirate and/or peripheral blood is preferably withdrawn from the patient without an anti-coagulant. A tray of the scaffold is provided and the bone marrow aspirate and/or peripheral blood is mixed directly with the scaffold. The mixture is then allowed to coagulate by the natural process of coagulation of the bone marrow aspirate and/or peripheral blood. In other words, no additional coagulating factors or agents are applied to the mixture. The coagulum mixture including the scaffold is then immediately used in the patient and applied at the surgical site, for example, for bone healing at the lateral gutters of the spine.

### Example 1: Ceramic-Based Scaffold

In this example, a ceramic-based scaffold includes particles of ceramic. The particles of ceramic include bioactive glass, tri-calcium phosphate, or a mixture thereof.

### Example 2: Collagen-Based Scaffold

In this example, a collagen-based scaffold includes fibers of collagen.

### Example 3: Ceramic and Collagen-Based Scaffold

In this example, a ceramic and collagen-based scaffold includes ceramic including bioactive glass, tri-calcium phosphate, or mixtures thereof and fibers of collagen.

The formulations may include as provided in the table below:

| | Example 1: Ceramic-Based Scaffold | Example 2: Collagen-Based Scaffold | Example 2: Collagen-Based Scaffold |
|---|---|---|---|
| Coagulum | 5 cc | 5 cc | 5 cc |
| Ceramic | 2.5 cc | n/a | 2.4 cc |
| Collagen Fibers | n/a | 3 cc | 0.6 cc |
| BMP dosage | 25 µg-1.5 mg/cc of composition | 25 µg-1.5 mg/cc of composition | 25 µg-1.5 mg/cc of composition |

The resulting compositions provide an autologous or allogenic carrier to deliver recombinant proteins for treating bone or cartilage regeneration. By adding additional osteoconductive and osteostimulative components to the coagulum, the regeneration process can be further mediated with mechanical support and cell attachment sites. The controlled release of the recombinant protein may help to reduce the side-effects previously seen with BMP-related technology.

Although the invention has been described in example embodiments, those skilled in the art will appreciate that various modifications may be made without departing from the spirit and scope of the invention. It is therefore to be understood that the inventions herein may be practiced other than as specifically described. Thus, the present embodiments should be considered in all respects as illustrative and not restrictive. Accordingly, it is intended that such changes and modifications fall within the scope of the present invention as defined by the claims appended hereto.

The invention could be defined inter alia by the following examples:
1. A composition for aiding tissue regeneration, the composition comprising: a coagulum comprising a hematic fluid; an exogenous recombinant protein; and ceramic particles, collagen fibers, or both.
2. A composition for aiding tissue regeneration, the composition comprising: a scaffold comprising one or more of ceramic particles, collagen fibers, and mixtures thereof; a coagulum comprising a hematic fluid dispersed through the scaffold; and an exogenous recombinant protein.
3. The composition of example 1, wherein the coagulum comprises one or more of platelets, red blood cells, white blood cells, plasma, bone marrow stroma, mesenchymal stem cells, osteoprogenitor cells, myelopoietic cells, erthyropoietic cells, and combination thereof.
4. The composition of example 1, wherein the coagulum comprises viable cells.
5. The composition of example 1, wherein the coagulum comprises coagulated bone marrow aspirate.
6. The composition of example 1, wherein the coagulum comprises coagulated blood.
7. The composition of example 1, wherein the recombinant protein comprises a bone morphogenetic protein.
8. The composition of example 1, wherein the recombinant protein is selected from the group consisting of BMP-2, BMP-4, BMP-6, BMP-7, heterodimers thereof, and combinations thereof.
9. The composition of example 1, wherein the ceramic particles are selected from the group consisting of bioactive glass, tri-calcium phosphate, and mixtures thereof.
10. The composition of example 1, wherein the ceramic particles comprise bioactive glass particles.
11. The composition of example 1, wherein the ceramic particles comprise tri-calcium phosphate particles.
12. The composition of example 1, wherein the ceramic particles have a particle size ranging from about 1 to 1000 µm.
13. The composition of example 1, wherein the ceramic particles have a pore size of about 100 to 500 µm with an overall porosity of about 60-80%.
14. A method of preparing an implantable composition for aiding tissue regeneration, the method comprising: obtaining a sample of hematic fluid from a patient without using an anti-coagulant to withdraw the sample; mixing the sample with an exogenous recombinant protein and ceramic particles, collagen fibers, or both to form a homogenous mixture; and allowing the homogenous mixture to naturally coagulate without adding a coagulation agent to form the implantable composition.
15. The method of example 14, wherein the recombinant protein and ceramic particles, collagen fibers, or both are mixed with the sample before the sample coagulates.
16. The method of example 14, wherein the sample of hematic fluid is bone marrow aspirate retrieved from the patient.
17. The method of example 14, wherein the sample of hematic fluid is peripheral blood retrieved from the patient.
18. The method of example 14, wherein the implantable composition is administered to the same patient from which the sample was obtained.
19. A method of promoting bone or wound healing in a mammal, the method comprising:
   providing a composition comprising a mixture of coagulum comprising a hematic fluid, an exogenous recombinant protein, and ceramic particles, collagen fibers, or both; and
   administering the composition into a target repair site to facilitate repair or regeneration of tissue at the target repair site.
20. The method of example 19, wherein the target repair site is an injury or defect in the spine and the tissue being regenerated is bone.

## Claims

1. A composition for aiding tissue regeneration, the composition comprising:
- a coagulum comprising a hematic fluid;
- an exogenous recombinant protein; and
- ceramic particles, collagen fibers, or both.

2. The composition of claim 1, wherein the coagulum comprises one or more of platelets, red blood cells, white blood cells, plasma, bone marrow stroma, mesenchymal stem cells, osteoprogenitor cells, myelopoietic cells, erthyropoietic cells, and combination thereof.

3. The composition of claim 1, wherein the coagulum comprises viable cells, or coagulated bone marrow aspirate or coagulated blood.

4. The composition of claim 1, wherein the recombinant protein comprises a bone morphogenetic protein.

5. The composition of claim 1, wherein the recombinant protein is selected from the group consisting of BMP-2, BMP-4, BMP-6, BMP-7, heterodimers thereof, and combinations thereof.

6. The composition of claim 1, wherein the ceramic particles are selected from the group consisting of bioactive glass, tri-calcium phosphate, and mixtures thereof.

7. The composition of claim 1, wherein the ceramic particles comprise bioactive glass particles.

8. The composition of claim 1, wherein the ceramic particles comprise tri-calcium phosphate particles.

9. The composition of claim 1, wherein the ceramic particles have a particle size ranging from about 1 to 1000 µm.

10. The composition of claim 1, wherein the ceramic particles have a pore size of about 100 to 500 µm with an overall porosity of about 60-80%.

11. A method of preparing an implantable composition for aiding tissue regeneration, the method comprising:
- mixing a sample of hematic fluid void of anti-coagulant with an exogenous recombinant protein and ceramic particles, collagen fibers, or both to form a homogenous mixture; and
- allowing the homogenous mixture to naturally coagulate without adding a coagulation agent to form the implantable composition.

12. The method of claim 11, wherein the recombinant protein and ceramic particles, collagen fibers, or both are mixed with the sample before the sample coagulates.

13. The method of claim 11, wherein the sample of hematic fluid is bone marrow aspirate.

14. The method of claim 11, wherein the sample of hematic fluid is peripheral blood.
